# EUROPEAN PATENT APPLICATION

(11) **EP 4 502 699 A1**
(43) Date of publication of application: **05.02.2025**
(21) Application number: 23189405.6
(22) Date of filing: 03.08.2023
(51) Int. Cl.: G02B 21/36, G06T 7/00

(54) **COMPUTER-BASED METHODS FOR ANALYZING A PLURALITY OF IMAGES, DIAGNOSTIC AND MEDICAL DEVICES, AND GUI**

(71) Applicant: Leica Microsystems CMS GmbH, 35578 Wetzlar (DE)
(72) Inventor: Wittke, Werner, 35578 Wetzlar (DE)
(74) Representative: Paustian & Partner Patentanwälte mbB

(57) **Abstract**

A first aspect of this disclosure is related to a method for analyzing images, comprising the steps:
- determining a first set of requirements;
- obtaining a plurality of images of a specimen from an imaging device, in particular in a microscope, based on the first set of requirements; - obtaining a second set of requirements;
- analyzing the specimen from the plurality of images based on the second set of requirements.

## Description

### Technical Field

This disclosure is related to computer-based methods for analyzing a plurality of images. Additionally diagnostic and or medical devices are disclosed that can be used in connection with the disclosed methods. Furthermore, GUIs for these embodiments described.

### Background

Biological, chemical, diagnostic, or medical analyses based on an image stream of a sample or specimen use specialized imaging techniques to examine and understand a structure, composition, and functionality of the object. These analyses can comprise, e.g. histological tissue studies for disease detection, fluorescence imaging of cellular components, or stain-free analyses. In chemical analyses, imaging spectrometers can capture e.g. spectral signatures to identify molecular compounds. Imaging analysis can improve our ability to accurately diagnose disease, study cellular interactions, and advance medical research, contributing to better treatments and a deeper understanding of biological processes.

A typical problem with these analyses is the enormous amount of data that must be analyzed to obtain insightful results. This can take a great deal of time and/or require large amounts of IT resources. Improvements are therefore desirable.

### Summary

An object of the present disclosure is improvement of image-based analyses.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of this disclosure is related to a method for analyzing images, comprising the steps:
- determining a first set of requirements;
- obtaining a plurality of images of a specimen from an imaging device, in particular in a microscope, based on the first set of requirements; - obtaining a second set of requirements;
- analyzing the specimen from the plurality of images based on the second set of requirements.

A first set of requirements can be obtained based on a user input and/or automatically. In particular, a first set of requirements can be configured based on a specific analysis type and/or based on a specific device type, with which the analysis should be performed. A first set of requirements can also be received and/or fetched from another device, in particular from a diagnostic/clinical/medical device and/or a communication device, such as a cloud. The first set of requirements governs the execution of the image acquisition. In other words, the first set of requirements governs the acquisition of information based on which a later analysis is performed. A first set of requirements can comprise one or more requirements.

Diagnostic and/or medical images can be acquired using different imaging devices, e.g. for one or more of the following image types:
- photography images, e.g. as used in light microscopy, phase contrast microscopy or fluorescence microscopy;
- X-ray images;
- computed tomography (CT);
- magnetic resonance imaging (MRI);
- ultrasound, positron emission tomography (PET).

Image acquisition involves capturing the images using the appropriate modality and ensuring proper image quality.

A second set of requirements can be obtained in one or more of the ways already described for a first set of requirements. A second set of requirements can comprise one or more requirements. The second set of requirements governs the analysis itself. It can comprise one or more requirements that are also comprised by the first set of requirements. Alternatively, the second set of requirements can comprise requirements that differ from the requirements of the first set of requirements.

An analyzing of the plurality of images based on the second set of requirements can comprise one or more analyses. Such an analysis can comprise a frame-by-frame analysis, in which each image is analyzed independently. Thereby, image analysis techniques, such as segmentation, feature extraction, and classification, etc. can be applied to each individual image. The results from each image analysis can be analyzed separately or combined to derive meaningful insights.

Additionally or alternatively, an analyzing of the plurality of images based on the second set of requirements can comprise a temporal analysis. Thereby, a stream of images provides temporal information, allowing for the analysis of changes and/or patterns over time. A temporal analysis can involve comparing consecutive frames to detect motion, track objects, identify temporal patterns, or quantify temporal dynamics. Techniques like optical flow, background subtraction, and tracking algorithms can be used for this purpose.

Additionally or alternatively an analyzing of the plurality of images based on the second set of requirements can comprise an event-detection-analysis. An event-detection-analysis can focus on identifying specific events and/or activities within the obtained images. This could involve recognizing predefined actions, abnormal events, and/or significant changes in the scene. Machine learning techniques, such as recurrent neural networks and/or hidden Markov models, can be employed for event detection in image sequences.

Additionally or alternatively an analyzing of the plurality of images based on the second set of requirements can comprise an object tracking. Object tracking can be configured to follow and locate specific objects of interest across multiple images. An object tracking can involve associating objects detected in one frame with their corresponding instances in subsequent frames, allowing for analysis of object trajectories, speed, interactions, or behavior over time. Tracking algorithms can be based on methods like Kalman filtering, particle filtering, and/or data association techniques.

Additionally or alternatively an analyzing of the plurality of images based on the second set of requirements can comprise an object tracking. A motion analysis in a stream of images (i.e. in the obtained images) can provide insights into dynamic events and activities. Techniques like optical flow analysis, background subtraction, and/or motion energy analysis can be used to extract motion information from the image stream. This information can be further analyzed to detect anomalies, characterize patterns of motion, or understand object dynamics.

Additionally or alternatively an analyzing of the plurality of images based on the second set of requirements can comprise a change detection. A change-detection-based analysis can focus on identifying differences and/or changes between consecutive frames. It can be used to detect and highlight regions of interest that have undergone changes, such as new objects, appearance/disappearance of objects, or alterations in the scene. Techniques like image differencing, image registration, or pixel-wise comparison can be employed for change detection.

Additionally or alternatively an analyzing of the plurality of images based on the second set of requirements can comprise an analysis based on a fusion of multiple images. Instead of analyzing a single image individually, multiple images can be fused or combined to extract higher-level information. Such an analysis can involve an image averaging, a background modeling, and/or a feature aggregation across image.

Additionally or alternatively an analyzing of the plurality of images based on the second set of requirements can comprise a real-time analysis. Real-time analyses can involve designing algorithms that can process and analyze images within the given time constraints and/or utilizing parallel processing of different analyses.

Based on a method according to the first aspect resources for analyses can be reduced.

A first aspect of this disclosure is related to a method for analyzing images, wherein the first set of requirements comprises one or more requirements related to a content of one or more images.

A content of an image can refer to the information, features, and/or visual elements present within the image. It can e.g. comprise objects, patterns, textures, colors, shapes, frequencies, functions, and/or structures that are visible in an image. A content-based requirement can relate to an image feature related to context in which an image or a plurality images is captured. In case of a microscope image this can be a morphological, (bio)chemical, and/or molecular biological context. One or more content-related parameters can be set as first requirements in particular dependent on the later analysis.

A content-based requirement can comprise a segmentation. Thereby, one or more images can be divided into meaningful regions or objects of interest. E.g. a segmentation can be used to identify and separate specific anatomical and/or morphological structures, lesions, cell organs, or abnormalities. Various algorithms, such as thresholding, region growing, edge detection, and clustering, can be employed for segmentation.

A content-based requirement can comprise a feature extraction. E.g. once a region of interest has been identified through segmentation, one or more relevant features can be extracted from those regions. Features could include geometric properties (e.g., size, shape, volume), texture characteristics, intensity statistics, or other quantitative measures that describe the structures, regions, and/or abnormalities within the image. A content-based requirement can comprise subcellular structures such as a mitochondrion, a cell core, a spindle apparatus, and/or other tissue structures.

A content-based requirement can comprise a feature extraction. An extracted feature can be utilized to classify and/or recognize a specific patterns, structures, or abnormalities within a diagnostic and/or medical image. A feature extraction cab be performed, e.g. by a machine learning algorithm, such as a convolutional neural network. Also classification algorithms, such as machine learning techniques (e.g., support vector machines, random forests, convolutional neural networks), can be employed to distinguish between different regions and/or identify specific conditions.

A content-based requirement can comprise a quantitative analysis. A quantitative analysis can involve measurement and/and numerical evaluation of specific parameters and/or characteristics within an image. It can include measurements of size, volume, density, intensity, and/or other quantitative metrics that aid in diagnosis, monitoring, or treatment planning.

A content-based requirements enables the imaging device to obtain only the images that are required for a specific analysis. This can reduce stress on the probe, e.g. phototoxicity, and save resources.

A first aspect of this disclosure is related to a method for analyzing images, wherein the first set of requirements comprises one or more of the following:
- a spatial position;
- an identification of a sample-related carrier;
- a time-related parameter;
- a wavelength-related parameter;
- an intensity difference;
- a movement parameter;
- a pattern.

A spatial position can be related to the translational x, y, z coordinates in a three-dimensional space of an analyzed subject matter, i.e. a probe in a microscope. These coordinates can represent a subject matter's lateral (horizontal) position along the x- and y- axes and its axial (vertical) position along the z axis. Thereby, a precise determination of an imaging field of a subject matter is possible. By knowing a subject matter's position, it can be ensured that the desired area or feature is being examined.

In a microscope, e.g. a spatial position of a specimen can be associated with the stage position, which determines the location of the probe relative to the fixed reference point of the microscope. A spatial position of an analyzed subject matter can be related to the focal plane of the microscope. The focal plane is the specific plane within subject matter (also "sample" or "specimen") that is in focus, while other planes may appear blurred. Accurate positioning of the specimen within the focal plane is essential for capturing clear and detailed images of the specimen and its surroundings. In some cases, multiple images, e.g. microscope images, may be acquired and stitched together to create a larger, composite image of the specimen or sample. The spatial position of a specimen can be associated with one or more overlapping regions between these images, ensuring accurate alignment and/and seamless integration of the specimen's data across different frames.

Microscope specimen can be associated with different coordinate systems, depending on the specific application or imaging technique. For instance, in fluorescence microscopy, specimen may be associated with a coordinate system of fluorescence channels and/or specific fluorophores used for labeling. Obtaining an image of a specimen depending on a spatial position within a relevant coordinate system helps in interpreting and analyzing the acquired data later on.

When working with multi-modal or multi-spectral imaging, a spatial position of a specimen can be associated with the registration of different image modalities and/or channels. Image registration involves aligning images acquired from different sources and/or imaging modalities to ensure accurate spatial correspondence. The probe's position can be utilized for registration purposes, enabling the fusion or overlay of different imaging data.

A requirement of a first set can be a time-related parameter. A time related parameter can be an exposure time. An exposure time refers to a duration during which an imagine device's sensor or detector is exposed to light during the image capture process. The appropriate exposure time is essential for achieving optimal image quality and avoiding overexposure or underexposure. Longer exposure times can capture more light and increase image brightness.

A time related parameter can be a frame rate. The frame rate determines the number of images captured per unit of time. It represents the temporal resolution of the image sequence and is typically expressed in frames per second (fps). Higher frame rates allow for the capture of fast-moving parts of a specimen and/or dynamic processes with more temporal detail.

A time related parameter can be related to a time-lapse imaging: Time-lapse imaging can involve capturing a sequence of images at specific time intervals over an extended period. It allows for the observation of time-dependent processes, changes, or interactions involving a subject matter to be analyzed. The time duration between each image in a time-lapse sequence can be predefined based on a desired temporal resolution and/or a dynamics of the observed phenomena.

A time related parameter can be associated with a speed and/or a rate at which the imaging device captures and processes images. Faster imaging speeds can be crucial for real-time and/or dynamic applications.

A time related parameter can be an exposure dwell time. For example in scanning microscopy techniques such as confocal microscopy or scanning electron microscopy (SEM), a probe is scanned by a beam. The exposure dwell time can refer to the duration that the beam spends at each pixel or point during the scanning process. The dwell time affects the signal-to-noise ratio, resolution, and image quality.

A time related parameter can be an acquisition time. An acquisition time is the total duration required to capture a single image and/or an entire image sequence. It can comprise all processes involved in image acquisition, including focusing, exposure, signal readout, and data transfer. Minimizing the acquisition time is important for capturing fast events or reducing the impact of motion artifacts.

A requirement of a first set can be a frequency. A frequency can refer to a frequency used by an imaging device to obtain images. For example, frequencies in near field microscopy can comprise optical frequencies and/or infrared frequencies.

A requirement of a first set can be an intensity difference. E.g. in a microscope image, an intensity difference can refer to variations and/or discrepancies in the brightness and/or intensity levels of different regions and/or features within the image. An intensity difference can represent a contrast and/or a distinction between areas of different light intensity. The intensity difference in a microscope image can arise from several factors, such as e.g. absorption or transmission characteristics of a specimen and/or refractive index variations. Furthermore, an intensity difference can be related to a fluorescence emission in fluorescence microscopy. A fluorescence emission can be related to fluorescent signals from specific regions and/or structures within the sample.

A requirement of a first set can be related to a movement parameter. A movement parameter can be related to a dynamic processes of the specimen, the imaging device, and/or the environment of the imaging device, which can be unveiled based on obtained images. For example, in live cell imaging, the movement of cells, organelles, and/or other cellular components can indicate cellular activities, migration, or response to stimuli. A movement-related parameter can be related to cell motility, flow analyses, particle tracking, tissue morphology, and time-lapse imaging. A movement parameter can also be related to mechanical properties of a specimen, such as stiffness, elasticity, or surface deformations.

A requirement of a first set can be related to a pattern, in particular a pre-defined pattern. A pattern of a specimen analyzed by an imaging device can provide information about a structure, an organization, and/or characteristics of the specimen.

Patterns can include regular structures, such as crystals, lattice arrangements, or repeating motifs, as well as irregular or random distributions of features. A presence of symmetrical patterns and/or symmetry breaking where patterns deviate from a certain symmetry, can provide insights into a sample's development, growth, or response to external factors. Crystallography-related parameters, such as used in polarized light microscopy and X-ray diffraction, can reveal the crystal lattice and crystallographic properties. Additional or alternatively, a pattern can relate to a cellular arrangement, surface textures, fractals and self-similarities, spatial distributions (in particular within fluorescence microscope) and/or biomolecular patterns, e.g. to identify biomolecules, such as proteins, DNA, and RNA, within cells and tissues.

A first aspect of this disclosure is related to a method for analyzing images, wherein the first set of requirements comprises one or more requirements related to a form of one or more images.

One or more form-related parameters can be set as first requirements in particular dependent on the later analysis. The acquired images can undergo preprocessing steps to enhance image quality, reduce noise, correct artifacts, and normalize image characteristics. Preprocessing techniques may include filtering, image registration, denoising, and image normalization. One or more of these pre-processing features can also be set as first requirements.

A first aspect of this disclosure is related to a method for analyzing images, wherein the first set of requirements comprises one or more of the following:
- an image size;
- an image aperture;
- an image resolution.

An image size can relate to an image form and/ or the x-, y-, z-dimensions of an image. An image size can also relate to focus, zooming, tilting, and rotation. A first requirement related to image size can additionally or alternatively be related to a detected interesting region, such as a structure, plus a relevant environment, in particular an environment directly next to an interesting region.

A contrast refers to the ability of the microscope to distinguish the specimen from its background and/or surrounding materials.

An aperture can relate to an aperture size and/or a depth of field. A depth of field indicates a thickness of the plane in focus. A larger depth of field allows for a greater part of the specimen to be in focus simultaneously, providing a more comprehensive view. An aperture can also relate to an illumination.

A first set requirement related to an image resolution can modulate a level of detail that can be observed. An image resolution can also relate to a magnification of the image. Higher magnification enables a closer examination of a specimen's features. Image resolution can also relate to a temporal resolution related to a rate in which images are obtained by the imaging device.

For the later analysis, an overall quality of the image in the context of the analysis is important. Therefore, first set requirements can also comprise parameters related to sharpness, clarity, color accuracy, and/or lack of artifacts and/or distortions.

A first aspect of this disclosure is related to a method for analyzing images, wherein the obtaining of the plurality of images comprises the step:
- storing a subset of images provided by an imaging system.

By storing a reduced image stream, less memory and faster data processing for the later analysis can be achieved.

Storage of images from an imaging device, in particular a microscope, can involves managing and/or preserving the image data for the later analysis. A storage can be related to a file format, such as TIFF and/or RAW formats. A storage can further depend on a predefined resolution for one or more images, in particular images of a reduced image stream can have different file and/or resolution formats. Additional or alternatively, a storage can further comprise metadata to one or more images of a reduced image stream. Metadata provides context and aids in image interpretation and analysis and in particular related information present during the capturing of an image. In particular, storage one or more storage parameters can be based on the first set of requirements and/or the second set of requirements.

A first aspect of this disclosure is related to a method for analyzing images, wherein the obtaining of the plurality of images comprises the step:
- controlling an imaging system for capturing images.

Controlling an imaging system, especially that of a microscope, involves can comprise control of various parameters, in particular parameters comprised and/or related to in the first set of requirements. A control of an imaging system can comprise a control of an objective selection, control of one or more parameters for focus adjustment, control of illumination settings, control of shutter speed and/or exposure time. Additionally or alternatively, control of an imaging device can comprise a control of a filter selection. In particular in fluorescence microscopy, selecting certain filters can allow for an isolation of specific fluorescence wavelengths and/or can minimize background noise. Controlling the image acquisition process, in particular triggering image capture, adjusting a frame rate for time-lapse imaging, and/or managing a total number of frames to be acquired can also be comprised by a control of an imaging system. In 3D imaging, controlling a microscope to capture a series of images at different focal planes can allow for Z-stack reconstruction. For fluorescence microscopy and multi-color imaging, controlling the system for capturing multiple channels or spectral bands can be performed by a controlling of an imaging system.

A first aspect of this disclosure is related to a method for analyzing images, wherein the obtaining of the plurality of images comprises an event-triggered obtaining of one or more images.

An event that can trigger the step 'obtaining a plurality of images' is tied to one or more of the first set requirements. In particular an event-triggered image acquisition can result in a timely non-equidistant acquisition of images. An event-triggered obtaining of one or more images can result in a selection and storage of a certain image from an image stream taken by an imaging device. Additionally or alternatively, an event-triggered obtaining of one or more images can also result in capturing an image by activating the imaging device. Thereby, an image acquisition may only happen when a requirement is met.

An event can trigger an automated time-lapse imaging such that images can be captured at pre-defined time intervals. This can be used for observing dynamic processes, such as cell growth or movement, over extended periods. An event can further trigger an obtaining of an image in case of a specific event, in particular an event related to the specimen to be analyzed, e.g. when a certain threshold of fluorescence intensity is reached in a live cell imaging experiment. Additionally or alternatively, an event can further trigger an obtaining of an image in case of external events, such as changes in temperature, pressure, or other environmental conditions. An event can further be defined by an image acquisition protocol. Depending on an analysis, in particular based on first and/or second set requirements, predefined protocols for capturing specific images can be in place and an imaging device can be programmed to follow these protocols, ensuring consistent and reproducible image acquisition.

A first aspect of this disclosure is related to a method for analyzing images, wherein the obtaining a plurality of images is based on a machine learning algorithm.

Different machine learning algorithms can be used, e.g. Convolutional Neural Networks (CNNs), in particular to execute image classification, object detection, and/or segmentation tasks. In microscopy, CNNs can be employed to identify and classify cells, organelles, and/or other structures of interest within the images. Additionally or alternatively, semantic segmentation can be used to classify each pixel in an image into specific classes and/or regions of interest. Convolutional neural networks with architectures like U-Net or DeepLab can be used for semantic segmentation tasks in microscopy. Additionally or alternatively, instance segmentation can be used to identify and/or differentiate individual instances of objects within an image. Region-based convolutional neural networks and/or similar architectures can be used for instance segmentation tasks in microscopy. Additionally or alternatively, object detection algorithms can locate and classify multiple objects of interest within an image. Furthermore recurrent neural networks (RNNs): RNNs and their variants, like Long Short-Term Memory (LSTM) networks, can be used for analyzing sequential data based on the obtained plurality of images. They can be applied in time-series microscopy data and/or videos, capturing temporal dependencies in cell behavior or other dynamic processes. Random forests and/or support vector machines (SVMs) can be used for image analysis, in particular to identify different regions in an image.

A first aspect of this disclosure is related to a method for analyzing images, wherein the machine learning algorithm is based on the first set of requirements and/or on the second set of requirements.

A result of a machine learning algorithm can satisfy a first set requirement. In this case, an obtaining of one or more images can be triggered automatically. For example, based on a CNN or an SVN, a pre-defined state of a specimen can be identified in a "pre-image", which is constantly taken of the specimen by an imaging device. Based on this event, the imaging device can be triggered to capture a "full image" (pre-image and full image may differ in lighting conditions). Alternatively, e.g. if full images are captured constantly/continuously, an image selection and an image storing can be triggered. In both preceding alternatives, only full images may be stored that meet the first set requirement monitored by the machine learning algorithm. Therefore, the machine learning algorithm can be executed under soft or hard real-time conditions.

A first aspect of this disclosure is related to a method for analyzing images, wherein the machine learning algorithm comprises a neural network, in particular a convolutional neuronal network.

Based on a neural network a monitoring of one or more first set requirements can be performed. A neural network can comprise a standard neural network, e.g. VGG and/or ResNET. VGG is a convolutional neural network architecture that is characterized by its use of small (3x3) convolutional filters and deep stacking of convolutional layers. ResNET is a deep convolutional neural network architecture that uses residual connections to enable training of very deep neural networks, up to hundreds of layers deep. In particular, a convolutional neural network can be used for efficient image processing. To determine events, states, and/or structures within a specimen a fully connected single or multilayer regression neural network can be used. A regression neural network can be a feedforward neural network, such as a multilayer perceptron. The regression neuronal network in particular can be configured to obtain its input from a convolutional neural network. Thereby, in particular a segmentation of one or more obtained images can be computed with high accuracy.

A first aspect of this disclosure is related to a method for analyzing images, wherein the step obtaining a plurality of images from an imaging device comprises:
- obtaining a plurality of images from a first device;
- obtaining a plurality of images from one or more further devices.

The obtaining of images from a first device and one or more further devices can be performed concurrently and/or sequentially. First and further devices can be, e.g., part of a distributed microscope system, connected via a network. The microscopes of the distributed system can be located on the same place or on different places and are coordinated to work together to capture images and/or data from a same and/or different specimen. This setup can allow a fast capture of relevant images for a later analysis.

A first aspect of this disclosure is related to a method for analyzing images, parallel to the obtaining a plurality of images of the specimen, comprising the step:
- controlling an influence on the specimen, e.g. a provision of a liquid and/or a mechanical pressure.

An influence of the specimen can be exerted by the imaging device and/or by an additional device. An additional device can be, e.g., a liquid handling device, that is configured to add a liquid to the specimen if a pre-defined condition is met. This condition can be, e.g., a first or second set requirement. Additionally or alternatively, an influence can be a mechanical/hydrodynamical influence, e.g. a pressure, a biochemical influence, e.g. an addition of an agent, and/or a molecular influence, e.g. a DNA probe inserted into a specimen.

A second aspect of this disclosure is related to a diagnostic and/or medical device, in particular a microscope, configured for:
- executing a method according to the first aspect and/or any of its embodiments; or
- receiving control information from a method according to the first aspect and/or any of its embodiments.

Such a device can be an imaging device. Additionally or alternatively, such a device can be an additional device that is configured to exert a predefined influence on a specimen according to the preceding embodiment of the first aspect. Additionally or alternatively, such a device can be a computation device that is configured to receive images about a specimen from an imaging device and/or control the imaging device and/or the additional device, which is configured to exert an influence on the specimen.

A third aspect of this disclosure is related to a graphical user interface (GUI), in particular for diagnostic and/or medical device,
configured to:
- define content-related and/or format-related requirements for an image acquisition;
- display one or more images according to the defined requirements;
- interface with a device according to the preceding aspect.

The GUI can be used to define one or more first set requirements for the acquisition of images of one or more specimen. For example, a user can provide a region of interest and the image device will be controlled based on this region of interest and will focus on this region for one or more further images. After analysis, results can be visualized and presented in a manner that are easily interpretable by medical professionals. Visualization techniques can include 2D and 3D rendering, multi-planar reconstructions, surface rendering, or other methods that effectively display the analyzed data.

In some cases, medical and/or diagnostic decision support systems can be visualized by the GUI to assist healthcare professionals in making accurate and informed diagnoses or treatment decisions. These systems can incorporate image analysis algorithms, clinical guidelines, patient data, and/to other relevant information to provide recommendations or assistance to medical practitioners.

A GUI can visualize a correlation function. Thereby, imaging information (e.g., microscope data, morphological data) can be linked to (bio)chemical data (e.g., proteins) and/or molecular biological data (e.g., information about RNA, DNA). By the GUI a user can also select which information should be correlated with the information from the images for an analysis.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 shows a set-up and workflow of an embodiment for analyzing specimen of the present disclosure.
Fig. 2 shows a flow chart of a method according to an embodiment of the present disclosure.
Fig. 3 shows a convolutional neural network configured for an embodiment of this disclosure.
Fig. 4 shows a microscope system as can be used for embodiments of this disclosure.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed description

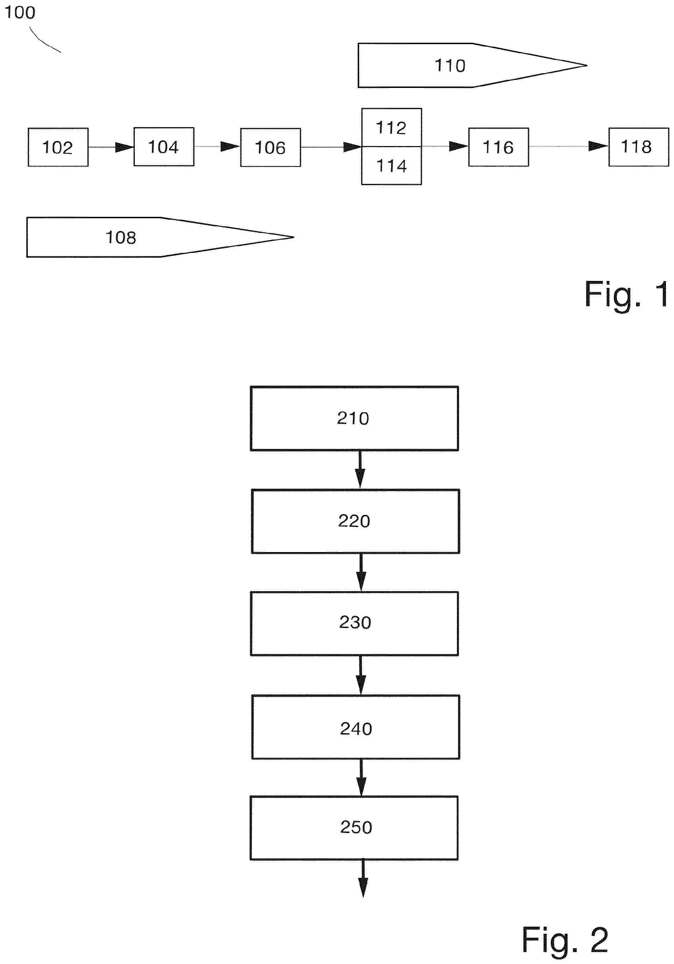

Fig. 1 shows a set-up and workflow of an embodiment 100 of the present disclosure. The experiment and therefore the generation of data starts with setting up and preparing the analysis by defining a first set of requirements 102, comprising e.g. a structure of an interesting region in the images that can be captured by a microscope for the experiment. A machine-learning-based detection (and selection) algorithm is trained on the first set of requirements 104. Alternatively a pre-trained algorithm can be used. This algorithm is configured to identify what type of data (structures, events, intensities, colors, movements, etc.) are required to perform the later analysis. The machine-learning algorithm automatically sets the necessary acquisition settings required to deliver images according to the first set of requirements.

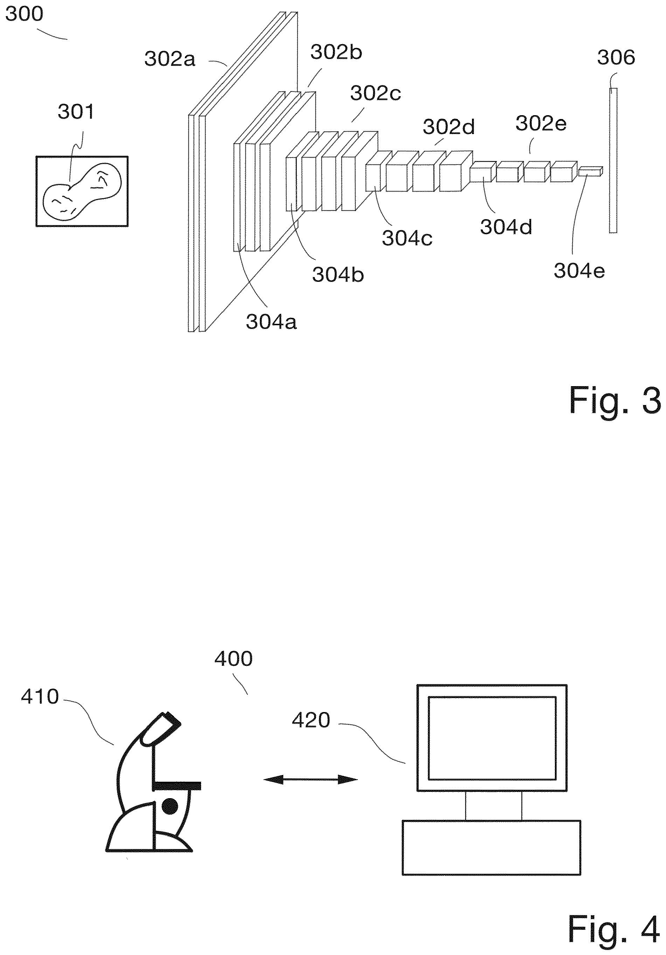

The machine-learning-based detection (and selection) algorithm can be based e.g. on a convolutional neural network (CNN) as described in Fig. 3. The CNN is trained to identify the region of interest in the images that are acquired by the microscope. The CNN can be trained and applied for fast recognition (in real-time) such that changes in a specimen can be identified shortly after their emergence. The machine-learning-algorithm is then interfaced with the imaging device 106, such that it can control an image acquisition according to the first set of requirements. The first three steps 102, 104, 106 constitute a pre-analysis configuration/set-up 108.

Thereafter, the configured image acquisition and data analysis 110 can be performed. Based on the first set requirements, the CNN controls the imaging device to only capture images that meet the first set requirements. This is done during step 112. All other images are not captured. This focusses the generated image stream to images that are valuable for the later analysis. In other words, by the conditional image acquisition process 112, a data reduction 114 is achieved.

Based on the reduced image data 112, 114, the analysis 116 is performed. The analysis is based on second requirements that differ from the first set requirements according to which the image selection was performed.

A graphical user interface (GUI) is configured to guide a user through the analysis workflow 118. The user can define the region of interest, e.g. based on existing, proposed examples, and this information is then used throughout the analysis for the CNN.

The analysis settings can be adjusted (automatically and/or by a user over a GUI) with respect to the images to be acquired. Settings such as S/N ratio, frame acquisition, number and "thickness" of z-steps (perpendicular to a focal plane), speed of imaging can be defined by the user and/or automatically adjusted, in particular based on the first set of requirements. Furthermore, parameters such as camera settings, time lapse settings, and/or positions settings can be defined.

Different machine-learning-based detection algorithms can be used for controlling the image acquisition. Different machine-learning-based detection algorithms can be trained based on different sets of first requirements. E.g. one detection algorithm can be trained to identify cell division and another detection algorithm can be trained to recognized certain cell structures. A suitable detection algorithm can be automatically selected based on the first set requirement(s).

Additionally or alternatively, a machine-learning detection algorithm can also be selected by the user over the GUI. This can in particular be helpful if there are more than one detection algorithm suitable for a specific analysis and/or for one first set of requirements. Therefore, the GUI can be configured to propose to the user one or more detection algorithms. A GUI can also be used for further training of the detection/selection algorithm. Additionally or alternatively, the GUI can be configured to display information about the selected image acquisition method (i.e. the selected detection/selection method).

Based on a reduced set of images on which the analysis is performed, first set and/or second set requirements can be visualized, e.g. a 6D visualization related to parameters such as xyz-coordinates of an interesting region, changes over time, wavelength-distribution of the interesting region, intensity differences in the interesting region and/or positions/IDs of a samples or sample carriers (e.g. different wells of a multi well plate). External biochemical and/or molecular biological (DNA, RNA, -omics data) information can be additionally integrated into and correlated with the analysis results, in particular a spatial context can be provided in which first set requirements, second set requirements and/or additional information is visualized to the user.

Based on the disclosed method, less images need to be taken for an analysis and a reduction of phototoxicity and a reduction of bleaching can be obtained. This can increase the quality of the obtained images and eventually the quality of the analysis based on the images. Unlike the current state of imaging/analysis systems, the embodiment provides a workflow and user experience that begins with the analysis, not with an (unconditional) imaging.

One advantage of the embodiment is a reduction of the large amount of data which would result from standard imaging acquisition methods. A reduced amount of data to be analyzed can also result in a reduction of processing resources needed and analysis time, in particular for long-term time-lapse experiments and/or of high content screening experiments. The described embodiment can reduce data to be stored in the main storage and/or in a long-term storage. Furthermore, the embodiment can free up space and processing load of a GPU and/or CPU of a computer that executes a method according to this embodiment. Feedback microscopy, autonomous imaging and analysis, 3D-visualization can be performed more efficient and smoother and allow correlation of information from imaging with data from other technologies, e.g. a spatial correlation of a structure and a function.

The embodiments described in the present disclosure may be used for various types of experiments such as experiments related to: Detection and analysis of structures/sample types/events/changes over time; detection and analysis of intracellular structures (e.g. mitotic spindle, mitochondria, nucleus, membrane, vesicle, cytoskeleton); detection and analysis of 2D cells (e.g. cell types, expression patterns, "positive/negative"); detection and analysis of 3D cell cultures (e.g. spheroids, organoids, formations, expressions, drug assays); detection and analysis of tissue sections (e.g. functional/morphological regions, patterns); detection and analysis of organisms (e.g. organs, protozoa, multicellular organisms, host-pathogens, model organisms); specimens on slides, dishes, well plates.

The embodiments described in this disclosure can be used for various types of analyses and/or detection methods, such as analyses/detection methods related to: Morphology/pathomorphology, rare events, cell cycle, cell division, movement/tracking/motility, intensity/change/increase/decrease/quantification, wavelength/color, counting of structures, interaction of structures, spheroid/organoid formation, gene expression, protein distribution, proliferation, development, apoptosis, live/dead analyses, effects of drugs/compounds.

The embodiments described in this disclosure can be used for various types of applications, such as applications related to: Intracellular transport mechanisms, molecular interaction and dynamics, signal transduction, regenerative medicine, stem cells, genomics, proteomics, cancer research and diagnostics, immunology, embryogenesis, neural development, organ development.

Fig. 2 shows a flow chart of a method 200 according to an embodiment of the present disclosure. In a first step 210 a first set of requirements is obtained. The first set of requirements defines the content and the form of the images to be captured. By capturing only images with a predefined content/form, an analysis based on the captured picture can be performed with less resources and can achieve better results. The first step requirements can be provided by a database, depending on the type of analysis that should be performed. Additionally or alternatively, the first set of requirements can be provided by a user over a graphical user interface (GUI).

In a second step 220 a second set of requirements is obtained. The second set of requirements defines according to which requirements the analysis should be performed. In many cases the requirements from the first set differ from the requirements of the second set. However, it can also be that at least some requirements of the first set of requirements are comprised in the second set of requirements. The second step requirements can be provided by a database, depending on the type of analysis that should be performed. Additionally or alternatively, the second set of requirements can be provided by a user over a GUI. The second step 220 has to be performed (at least partly) before the analysis commences. It can be performed after, before, or parallel to the first step 210 and/or the third step 230.

In a third step 230 an imaging device is controlled to capture images that meet one or more, in particular all, of the first set of requirements. The imaging device may be a microscope, or another imaging device configured to observe a sample, specimen, and/or other object that is the subject of subsequent analysis.

In a fourth step 240 the obtained images are analyzed according to the second set of requirements. This step can be started after a first image has been captured and provided for analysis. Afterwards the third step 230 and the fourth step 240 can be performed concurrently unless sufficient image information has been obtained.

In a fifth step 250, the analyzed data are visualized for a user via the GUI. Thereby, in particular, the results of the analysis are illustrated together with information related to one or more requirement of the first and/or second set of requirement and/or with additional information. The GUI can be present during the whole process.

Fig. 3 illustrates a neuronal network for a method according to an embodiment of this disclosure. The neural network 300 is configured determine a region of interested with an image 301 of a specimen where a cell division takes place. The neural network identify not only images 301 that comprise a region of interest, but also the images that do not comprise a region of interest. The latter are not captured/saved as images for the analysis.

The neural network 300 can be a standard type of convolutional neuronal network ("VGG16"). An entry layer comprises two convolutional layers 302a with a size of 224x224x64. A first hidden layer comprises a max pooling layer 304a that samples the initial image down to a size of 112x112x128 by using a 3x3 receptive field. The first hidden layer furthermore comprises two convolutional layers 302b. A second hidden layer performs a downsampling to 56x56x256 by a max pooling layer 304b and is followed by three convolutional layers 302c. A third hidden layer performs a downsampling to 28x28x512 by a max pooling layer 304c and is followed by three convolutional layers 302d. A fourth hidden layer performs a downsampling to 14x14x512 by a max pooling layer 304d and is followed by three convolutional layers 302e. A final max pooling layer 304e performs a further downsampling to 7x7x512. This layer is connected to a single 8x1x1 fully connected layer 306 for a regression analysis.

The neural network 300 is trained with discrete frames from a video of with a respective analysis type as input. The training data can comprise labelled regions of interests, where a cell division takes place. Labels are gathered from human labeling. During training, the neural network is optimized via supervised back-propagation to maximize an intersection over union of the predicted bounding boxes for regions of interest and human gathered labels for each video frame.

Some embodiments relate to a microscope comprising a system as described in connection with one or more of the Figs. 1 to 3. Alternatively, a microscope may be part of or connected to a system as described in connection with one or more of the Figs. 1 to 3. Fig. 4 shows a schematic illustration of a system 400 configured to perform a method described herein. The system 400 comprises a microscope 410 and a computer system 420. The microscope 410 is configured to take images and is connected to the computer system 420. The computer system 420 is configured to execute at least a part of a method described herein. The computer system 420 may be configured to execute a machine learning algorithm. The computer system 420 and microscope 410 may be separate entities but can also be integrated together in one common housing. The computer system 420 may be part of a central processing system of the microscope 410 and/or the computer system 420 may be part of a subcomponent of the microscope 410, such as a sensor, an actor, a camera or an illumination unit, etc. of the microscope 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 100: method for analyzing specimen
- 102: defining first requirements
- 104: training machine learning algorithm
- 106: interfacing ML algorithm with selected imaging device
- 108: configurating analysis
- 110: obtaining images and analysis
- 112: performing selected acquisition
- 114: reducing data
- 116: analyzing reduced data
- 118: visualizing results
- 200: method flow chart
- 210: obtaining of first set of requirements
- 220: obtaining of second set of requirements
- 230: controlling imaging device
- 240: analyzing images
- 250: visualizing data
- 300: CNN for image analysis
- 301: image
- 302a: convolutional layers of entry layer
- 302b: convolutional layers of first hidden layer
- 302c: convolutional layers of second hidden layer
- 302d: convolutional layers of third hidden layer
- 302e: convolutional layers of third hidden layer
- 304a: max pooling layer of first hidden layer
- 304b: max pooling layer of second hidden layer
- 304c: max pooling layer of second hidden layer
- 304d: max pooling layer of second hidden layer
- 304e: final max pooling layer
- 306: fully connected layer
- 400: analysis system
- 410: microscope
- 420: computer

## Claims

1. A computer-based method for analyzing a plurality of images, in particular of diagnostic and/or medical images,
comprising the steps:
- determining a first set of requirements (102);
- obtaining a plurality of images of a specimen from an imaging device, in particular in a microscope, based on the first set of requirements (112);
- obtaining a second set of requirements;
- analyzing the specimen from the plurality of images based on the second set of requirements (116).

2. The method according to the preceding claim,
wherein the first set of requirements (102) comprises one or more requirements related to a content of one or more images.

3. The method according to the preceding claim,
wherein the first set of requirements (102) comprises one or more of the following:
- a spatial position;
- an identification of a sample-related carrier;
- a time-related parameter;
- a wavelength-related parameter;
- an intensity difference;
- a movement parameter;
- a pattern.

4. The method according to one of the preceding claims,
wherein the first set of requirements (102) comprises one or more requirements related to a form of one or more images.

5. The method according to the preceding claim,
wherein the first set of requirements (102) comprises one or more of the following:
- an image size;
- an image aperture;
- an image resolution.

6. The method according to one of the preceding claims,
wherein the obtaining of the plurality of images (112) comprises the step:
- storing a subset of images provided by an imaging system.

7. The method according to one of the preceding claims,
wherein the obtaining of the plurality of images (112) comprises the step:
- controlling an imaging system for capturing images.

8. The method according to one of the two preceding claims,
wherein the obtaining of the plurality of images (112) comprises an event-triggered obtaining of one or more images.

9. The method according to one of the preceding claims,
wherein the obtaining a plurality of images (112) is based on a machine learning algorithm.

10. The method according to the preceding claim,
wherein the machine learning algorithm is based on the first set of requirements (102) and/or on the second set of requirements.

11. The method according to the two preceding claims,
wherein the machine learning algorithm comprises a neural network (300), in particular a convolutional neuronal network.

12. The method according to one of the preceding claims,
wherein the step obtaining a plurality of images from an imaging device comprises:
- obtaining a plurality of images from a first device;
- obtaining a plurality of images from one or more further devices.

13. The method according to one of the preceding claims,
parallel to the obtaining a plurality of images of the specimen, comprising the step:
- controlling an influence on the specimen, e.g. a provision of a liquid and/or a mechanical pressure.

14. An imaging device (410), in particular a microscope,
configured for:
- executing a method (200) according to one of the preceding claims; or
- receiving control information from a method (220) according to one of the preceding claims.

15. A graphical user interface,
configured to:
- define content-related and/or format-related requirements (102) for an image acquisition;
- display one or more images according to the defined requirements (118);
- interface with a device according to the preceding claim (106).
